(19) Europäisches Patentamt

European Patent Office

Office européen des brevets

(11) **EP 0 797 966 A1**

## (12) EUROPEAN PATENT APPLICATION

(43) Date of publication:
01.10.1997 Bulletin 1997/40

(21) Application number: 96105021.8

(22) Date of filing: 29.03.1996

(51) Int. Cl.⁶: **A61F 13/15**, A61L 15/42,
A61L 15/60

(84) Designated Contracting States:
AT BE CH DE DK ES FR GB GR IE IT LI LU NL PT
SE

(71) Applicant: **THE PROCTER & GAMBLE COMPANY**
**Cincinnati, Ohio 45202 (US)**

(72) Inventors:
• **Palumbo, Gianfranco**
**61352 Bad Homburg (DE)**

• **Schmidt, Mattias**
**65510 Idstein (DE)**

(74) Representative: **Canonici, Jean-Jacques et al**
**Procter & Gamble European Service GmbH,**
**Sulzbacher Strasse 40-50**
**65824 Schwalbach am Taunus (DE)**

### (54) Collapsed superabsorbent foams

(57) A porous material for absorption of aqueous body fluids comprising superabsorbent polymer, which can swell upon absorption of aqueous liquids, and which forms a porous structure having a surface-to-mass ratio of at least 0.2m2/g, characterized in that the pores of said porous structure are collapsed to less than a third of their expanded volume prior upon wetting with said aqueous body liquids; and in that the pores expand upon wetting with aqueous liquids. The collapsed material can be in particulate or in a sheet like form.

EP 0 797 966 A1

**Description**

FIELD OF THE INVENTION

This invention relates to disposable absorbent articles, such as diapers, adult incontinence products, sanitary napkins and more particularly to disposable absorbent articles which have the capacity to retain aqueous body excrement's such as urine or menstrual fluids.

BACKGROUND OF THE INVENTION

Disposable, absorbent articles such as diapers, incontinence articles, sanitary towels, training pants and the like are well know in the art. Typically, disposable absorbent articles comprise a liquid pervious topsheet that faces the wearers body, a liquid impervious backsheet that faces the wearers clothing, and an absorbent core interposed between the liquid previous topsheet and the backsheet. The absorbent core must often be capable of absorbing and handling relatively large volumes of fluid like urine or other exudates discharged from the body of the wearer. The absorbent core needs to be capable of acquiring, distributing, and storing discharges initially deposited onto the absorbent article. Preferably the design of the absorbent core is such that the core acquires the discharges substantially immediately after they have been deposited on the topsheet of the absorbent article, with the intention that the discharges do not accumulate on or run off the surface of the topsheet, since this may result in inefficient fluid containment by the absorbent article which may lead to wetting of outer garments and discomfort for the wearer.

Numerous attempts have been made to address absorption of body fluids in absorbent members, with regard to improving acquisition of the fluids in the absorbent article, with regard to improving the distribution of such fluids throughout the absorbent article, but also with regard to providing improved ultimate storage of the fluid.

Several patent publications deal with improvements of fluid handling performance by adding specially treated cellulosic material. For example US patent 4 898 642 of Moore et al. discloses special twisted, chemically stiffened cellulosic fibres and absorbent structures made therefrom, and EP 0 640 330 of Bewick-Sonntag et al. discloses the use of such fibres in a specific arrangement with specific superabsorbent materials.

EP 0 312 118 (Meyer) discloses an absorbent article with a fibrous topsheet with larger pores than the pores of the underlying transport layer, which in turn has lager pores than the underlying absorbent body. Further, the transport layer has to have a hydrophilicity which is less than the one of the absorbent core, and may generally be characterised as being substantially hydrophobic.

In particular the use of superabsorbent materials (also called Absorbent Gelling Materials, or hydrogel materials) in such articles has gained broad usage. Superabsorbent materials are polymeric materials capable of absorbing large quantities of fluids, such as urine, and retaining such absorbed fluids under in use conditions.

Recent attempts have been made in the art to provide absorbent gelling materials which have the ability to swell against pressure. The alleged advantage in that the absorbent gelling materials absorb fluid under actual pressures exerted by the body during use. Yet other teachings in the art provide absorbent gelling materials having a particular free swell rate and absorbency under load. Alleged advantages of such absorbent gelling materials are lower volume and mass with approximately the same absorbent capacity, the ability to rapidly absorb a discharged liquid under pressures typically encountered during use, and the ability to retain the absorbed liquid under pressures typically encountered during use.

Examples of such attempts in the prior art include U.S. Patents 5,147,343 issued September 15, 1992 to Kellenberger and 5,149,335 issued September 22, 1992 to Kellenberger et al.

In order to enhance fluid handling properties of the absorbent structure, a number of attempts have been disclosed to increase the absorption speed of the superabsorbent material.

US-A-5.154.713 (Lind et al) discloses particulate superabsorbent materials having pores introduced during polymerisation of the material by e.g. moderate foaming the polymer by e.g. $CO_2$ release during polymerisation. After this, the polymer is treated in a conventional manner, i.e. it could be chopped, dried, finely ground, sieved (if necessary) and (optionally) surface crosslinked. This results in particulate superabsorbent materials looking macroscopically identical to untreated polymers, but showing in microscopic view of the surface of the particles small "Swiss-cheese-" like pores. The effect is a moderate increase in absorbency speed at a somewhat compromised gel strength.

US-A-5.149.334 (Berg et al.) disclose absorbent members comprising interparticle crosslinked aggregates which are made by agglomerating particles of a relatively small particle size and which remain aggregated even upon wetting due to this interparticle crosslinking. This results in relatively high density particulate structures with relatively few particles per aggregate structure. US-A-5.124.188 (Roe et al.) apply the same principle to create interparticle crosslinked macrostructures, such as in a sheet form, i.e. with relatively many particles per aggregate structure. For both approaches, the result is an increased absorption speed, but - unless high amounts of plasticiser such as glycerol and/or water are used - also a relatively stiff structure.

WO-95/22357 (Hayashi et al. ) disclose a process to create an increased specific particle surface, in submitting partly swollen superabsorbent particles to a free drying process, whereby the particles "explode" and form irregular structures, with a high surface to mass ratio, but with at best equal brittleness to conventional superabsorbent particles.

All of these absorbent elements have in common, that they result in relatively stiff structures.

An approach to provide high absorbency soft structures is to use superabsorbent fibres. Generally, such fibres are described by Bourland (US-A-4.855.179), or commercially available from Technical Absorbents Ltd, UK, or Camelot, US. Noel et al (EP-B-0 565 606) describe the use of such materials in absorbent structures. The superabsorbent fibres are comprised in low density webs, such as nonwoven structures of densities in the range of typically no more than 0.3g/cm3. Whilst such materials do have a good softness, and also exhibit relatively large surface to mass rations, their low density poses a disadvantage with respect to logistic difficulties upon production, and also with respect to high bulk of the resulting Absorbent article.

Further, US-A-5.338.766 (van Phan ) discloses open cell foam materials with the walls / struts of the foam being superabsorbent polymer. With only moderate amounts of plasticiser (such as water at a level of less than about 30% and preferably less than 15% by weight), the result is a highly porous, low density superabsorbent material with an increased absorption speed. Whilst this foam material is of low stiffness (or better softness), it carries essentially the same disadvantages as the low density web structures made from superabsorbent fibres.

An approach to reduce the dry volume of absorbent materials has been disclosed by Dyer (US-A-5.387.207). It is concerned with an absorbent foam made by High Internal Phase Emulsion polymerization process to form non-swellable polymers whereby the absorbent liquids are retained by capillary absorption forces in the pores of the foam. Such foams are then treated such that the pore volume collapse and the foams have about 10% to 20% of their initial volume. This small volume is maintained up until it is in contact with absorbed fluids (like water), when it increases to the initial volume, now providing absorbency in these pores. However, in such structures a balance of absorbency and stiffness properties has to be met: soft and very pliable structures are more prone to the undesired "squeeze out" effect of liquid being released upon pressure (which when occurring localised can reach relatively high values during use); relatively stiff structures are less prone to this effect, but less preferred for the lack of softness.

Hence it is an object of the invention to provide materials which offer a better balance of the properties of softness, absorbent capacity and absorbency speed.

It is a further object to provide such materials in either particulate form, or in macrostructures such as sheet forms.

It is further an object of this invention to provide absorbent structures and articles comprising such improved materials.

BRIEF SUMMARY OF THE INVENTION

The invention is concerned with a porous material for absorption of aqueous body fluids comprising superabsorbent polymer, which can swell upon absorption of aqueous liquids, and which forms a porous structure having a surface-to-mass ratio of at least 0.2m2/g, characterized in that the pores of said porous structure are collapsed to less than a third of their expanded volume prior upon wetting with said aqueous body liquids; and in that the pores expand upon wetting with aqueous liquids. The collapsed material can be in particulate or in a sheet like form.

BRIEF DESCRIPTION OF THE DRAWING

While the Specification concludes with claims pointing out and distinctly claiming the present invention, it is believed the same will be better understood by the following drawings taken in conjunction with the accompanying Specification wherein like components are given the same reference number and:

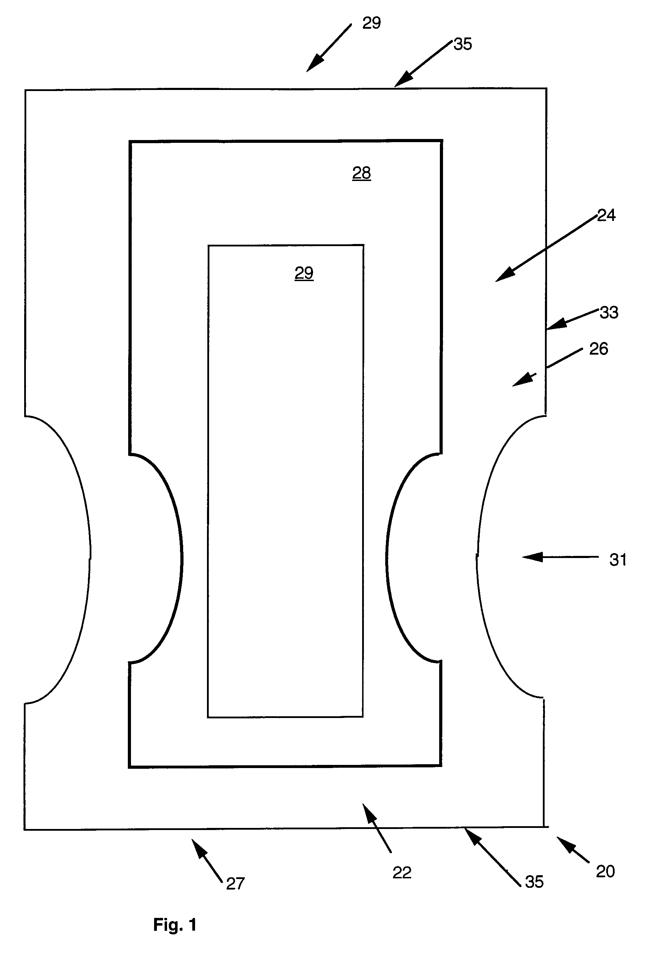

Fig.1 shows a Disposable Absorbent Article;

DETAILED DESCRIPTION OF THE INVENTION

Absorbent Articles

As used herein, the term "absorbent articles" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner).

An absorbent article generally comprises

- an absorbent core (which may consist of sub-structures);

- a fluid pervious topsheet;
- a fluid impervious backsheet;
- optionally further features like closure elements or elastification.

A specific embodiment of an absorbent article of the present invention is the disposable absorbent article, diaper 20, shown in Figure 1. As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments, and the like.

Figure 1 is a plan view of the diaper 20 in its flat-out, uncontracted state (i.e. with elastic induced contraction pulled out) with portions of the structure being cut-away to more clearly show the construction of the diaper 20 and with the portion of the diaper 20 which faces or contacts the wearer, the inner surface, oriented towards the viewer. As shown in Figure 1, the diaper 20 preferably comprises a liquid pervious topsheet 24; a liquid impervious backsheet 26 joined with the topsheet 24; an absorbent core 28 positioned between the topsheet 24 and the backsheet 26.

The diaper 20 is shown in Figure 1 to have a first waist region 27 juxtaposed with the front of the wearer while the diaper 20 is being worn, a second waist region 29 opposed to the first waist region 27 and juxtaposed with the back of the wearer while the diaper 20 is being worn, a crotch region 31 positioned between the first waist region 27 and the second waist region 29, and a periphery which is defined by the outer edges of the diaper 20 in which the longitudinal edges are designated 33 and the end edges are designated 35. The inner surface of the diaper 20 comprises that portion of the diaper 20 which is adjacent to the wearer's body during use (i.e., the inner surface generally is formed by at least a portion of the first topsheet 24 and other components joined to the first topsheet 24). The outer surface comprises that portion of the diaper 20 which is positioned away from the wearer's body (i.e., the outer surface generally is formed by at least a portion of the backsheet 26 and other components joined to the backsheet 26) during use.

Figure 1 shows a preferred embodiment of the diaper 20 in which the topsheet 24 and the backsheet 26 have length and width dimensions generally larger than those of the absorbent core 28. The topsheet 24 and the backsheet 26 extend beyond the edges of the absorbent core 28 to thereby form the periphery 22 of the diaper 20. While the topsheet 24, the backsheet 26, and the absorbent core 28 may be assembled in a variety of well known configurations, preferred diaper configurations are described generally in U.S. Patent 3,860,003 entitled "Contractable Side Portions for Disposable Diaper" which issued to Kenneth B. Buell on January 14, 1975; and U.S. Patent Application Serial No. 07/715,152, allowed, "Absorbent Article With Dynamic Elastic Waist Feature Having A Predisposed Resilient Flexural Hinge", Kenneth B. Buell et al. filed June 13, 1991.

The backsheet 26 is positioned adjacent the garment surface of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as bed-sheets and undergarments. The backsheet 26 may thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm to about 0.051 mm. Particularly preferred materials for the backsheet include RR8220 blown films and RR5475 cast films as manufactured by Tredegar Industries, Inc. of Terre Haute, IN, US. The backsheet 26 is preferably embossed and/or matte finished to provide a more clothlike appearance. Further, the backsheet 26 may permit vapours to escape from the absorbent core 28 (i.e., breathable) while still preventing exudates from passing through the backsheet 26.

The topsheet 24 is positioned adjacent the body surface of the absorbent core 28 and is preferably joined thereto and to the backsheet 26 by attachment means (not shown) such as those well known in the art. Suitable attachment means are described with respect to joining the backsheet 26 to the absorbent core 28. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

Generally, the topsheet 24 is compliant, soft feeling, and non-irritating to the wearer's skin. Further, the topsheet 24 is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques which may be used to manufacture the topsheet 24. For example, the topsheet 24 may be a nonwoven web of fibres spunbonded, carded, wet-laid, meltblown, hydroentangled, combinations of the above, or the like.

The diaper 20 may further comprise elasticised leg cuffs (not shown) which provide improved containment of liquids and other body exudates. Each elasticised leg cuff may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 describes a disposable diaper 20 which provides a contractible leg opening having a side flap and one or more elastic members to provide an elasticised leg cuff (gasketing cuff). U.S. Patent 4,909,803 entitled "Disposable Absorbent Article Having Elasticised Flaps" issued to Aziz et al. on March 20, 1990, describes a disposable diaper 20 having "stand-up" elasticized flaps (barrier cuffs) to improve the containment of the leg regions. Commonly assigned U.S. Patent 4,695,278 entitled "Absorbent Article Having Dual Cuffs" issued to Lawson on September 22, 1987, describes a disposable diaper 20 having dual cuffs including a gasketing cuff and a barrier cuff.

The diaper 20 preferably further comprises an elastic waist feature (not shown) that provides improved fit and containment. The elastic waist feature is that portion or zone of the diaper 20 which is intended to elastically expand and contract to dynamically fit the wearer's waist. The elastic waist feature at least extends longitudinally outwardly from at least one of the waist edges of the absorbent core 28 and generally forms at least a portion of the end edge of the diaper 20. Disposable diapers are generally constructed so as to have two elastic waist features, one positioned in the first waist region 27 and one positioned in the second waist region 29, although diapers can be constructed with a single elastic waist feature. Further, while the elastic waist feature or any of its constituent elements can comprise a separate element affixed to the diaper 20, the elastic waist feature is preferably constructed as an extension of other elements of the diaper 20 such as the backsheet 26 or the first topsheet 24, preferably both the backsheet 26 and the first topsheet 24. The elasticized waistband may be constructed in a number of different configurations including those described in U.S. Patent 4,515,595 issued to Kievit et al. on May 7, 1985 and the above referenced U.S. Patent Application Serial No 07/715,152; each of these references being incorporated herein by reference.

The diaper 20 also comprises a fastening system (not shown) which forms a side closure which maintains the first waist region 27 and the second waist region 29 in an overlapping configuration such that lateral tensions are maintained around the circumference of the diaper 20 to maintain the diaper 20 on the wearer. Exemplary fastening systems are disclosed in U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; commonly assigned U.S. Patent 4,946,527 entitled "Pressure-Sensitive Adhesive Fastener And Method of Making Same" issued to Battrell on August 7, 1990; commonly assigned U.S. Patent 3,848,594 entitled "Tape Fastening System for Disposable Diaper" issued to Buell on November 19, 1974; commonly assigned U.S. Patent B1 4,662,875 entitled "Absorbent Article" issued to Hirotsu et al. on May 5, 1987; and the hereinbefore referenced U.S. Patent Application 07/715,152; each of which is incorporated herein by reference.

The diaper 20 is preferably applied to a wearer by positioning one of the waist regions, preferably the second waist region 29, under the wearer's back and drawing the remainder of the diaper 20 between the wearer's legs so that the other waist region, preferably the first waist region 27, is positioned across the front of the wearer. The tape tabs of the fastening system are then released from the release portion. The diaper then wraps the elasticised side panel around the wearer, while still grasping the tab portion. The fastening system is secured to the outer surface of the diaper 20 to effect two side closure.

In general terms, the absorbent core 28 should be non-irritating to the wearer's skin, and capable of absorbing and retaining liquids such as urine and/or other body exudates. As shown in Figure 1, the absorbent core 28 has a garment surface, a body surface, side edges, and waist edges. The absorbent core 28 may be manufactured in a wide variety of sizes and shapes (e.g., rectangular, hourglass, "T"-shaped, asymmetric, etc.) and might comprise in addition to the materials of the current invention a wide variety of liquid-absorbent materials commonly used in disposable diapers and other absorbent articles such as - but not limited to - comminuted wood pulp which is generally referred to as airfelt; meltblown polymers including coform; chemically stiffened, modified or cross-linked cellulosic fibres; tissue including tissue wraps and tissue laminates.

The configuration and construction of the absorbent core 28 may also be varied (e.g., the absorbent core 28 may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the diaper 20. Further, the size and absorbent capacity of the absorbent core 28 may be varied to accommodate wearers ranging from infants through adults.

Exemplary absorbent structures for use as the absorbent core 28 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989; Also EP 0 640 330 of Bewick-Sonntag et al.; US 5 180 622 (Berg et al.); US 5 102 597 (Roe et al.) disclose structures which can be used to incorporate materials according to the present invention, which will now be explained in more detail.

The current invention concerns collapsible superabsorbent materials with a high surface-to-mass ratio which are in a collapsed state until - during use - they are in contact with body fluids such as urine or menstrual fluid. This provides additional benefits versus prior art materials. In particular, it allows to provide large amounts of absorbent capacity without compromising on bulk of the article nor on good fluid handling properties, especially with fast fluid pick up into the superabsorbent material as ultimate storage material for the fluids. Further, in particular for sheet like structures, the additional feature of flexibility ensures the absence of negatives as might be introduced through too stiff materials.

Materials according to the current invention can be made by starting from chemicals as for conventional superabsorbent materials.

These hydrogel-forming absorbent polymers preferably have a multiplicity of anionic, functional groups, such as sulfonic acid, and more typically carboxy, groups. Examples of polymers suitable for use herein include those which are prepared from polymerizable, unsaturated, acid-containing monomers. Thus, such monomers include the olefinically unsaturated acids and anhydrides that contain at least one carbon to carbon olefinic double bond. More specifically, these monomers can be selected from olefinically unsaturated carboxylic acids and acid anhydrides, olefinically unsaturated sulfonic acids, and mixtures thereof.

Some non-acid monomers can also be included, usually in minor amounts, in preparing the hydrogel-forming absorbent polymers herein. Such non-acid monomers can include, for example, the water-soluble or water-dispersible esters of the acid-containing monomers, as well as monomers that contain no carboxylic or sulfonic acid groups at all. Optional non-acid monomers can thus include monomers containing the following types of functional groups: carboxylic acid or sulfonic acid esters, hydroxyl groups, amide-groups, amino groups, nitrile groups and quaternary ammonium salt groups. These non-acid monomers are well-known materials and are described in greater detail, for example, in U.S. Patent 4,076,663 (Masuda et al), issued February 28, 1978, and in U.S. Patent 4,062,817 (Westerman), issued December 13, 1977, both of which are incorporated by reference.

Olefinically unsaturated carboxylic acid and carboxylic acid anhydride monomers include the acrylic acids typified by acrylic acid itself, methacrylic acid, ethacrylic acid, -chloroacrylic acid, a-cyanoacrylic acid, -methylacrylic acid (crotonic acid), -phenylacrylic acid, -acryloxypropionic acid, sorbic acid, -chlorosorbic acid, angelic acid, cinnamic acid, p-chlorocinnamic acid, -sterylacrylic acid, itaconic acid, citroconic acid, mesaconic acid, glutaconic acid, aconitic acid, maleic acid, fumaric acid, tricarboxyethylene and maleic acid anhydride.

Olefinically unsaturated sulfonic acid monomers include aliphatic or aromatic vinyl sulfonic acids such as vinylsulfonic acid, allyl sulfonic acid, vinyl toluene sulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-methacryloxypropyl sulfonic acid and 2-acrylamide-2-methylpropane sulfonic acid.

Preferred hydrogel-forming absorbent polymers for use in the present invention contain carboxy groups. These polymers include hydrolyzed starch-acrylonitrile graft copolymers, partially neutralized starch-acrylonitrile graft copolymers, starch-acrylic acid graft copolymers, partially neutralized starch-acrylic acid graft copolymers, saponified vinyl acetate-acrylic ester copolymers, hydrolyzed acrylonitrile or acrylamide copolymers, slightly network crosslinked polymers of any of the foregoing copolymers, partially neutralized polyacrylic acid, and slightly network crosslinked polymers of partially neutralized polyacrylic acid. These polymers can be used either solely or in the form of a mixture of two or more different polymers. Examples of these polymer materials are disclosed in U.S. Patent 3,661,875, U.S. Patent 4,076,663, U.S. Patent 4,093,776, U.S. Patent 4,666,983, and U.S. Patent 4,734,478.

As described above, the hydrogel-forming absorbent polymers are preferably slightly network crosslinked. Network crosslinking serves to render the polymer substantially water-insoluble and, in part, determines the absorptive capacity and extractable polymer content characteristics superabsorbent material. Processes for network crosslinking the polymers and typical network crosslinking agents are described in greater detail in the hereinbefore-referenced U.S. Patent 4,076,663, and in DE-A-4020780 (Dahmen). Also, the crosslinker type and the crosslinking density density can vary throughout the material, in particular when applying different and/or additional crosslinking to the surface of a particulate material, but also to different regions in a sheet like macrostructure.

Teabag Centrifuge Capacity Test

The Teabag Centrifuge Capacity test measures the Teabag Centrifuge Capacity values, which are a measure of the retention of liquids in the gelling materials (super absorber) at hydrostatic pressure.

The superabsorbent material is placed within a "teabag", immersed in a 0.9 % by weight sodium chloride solution for 20 minutes, and then centrifuged for 3 minutes. The ratio of the retained liquid weight to the initial weight of the dry superabsorbent material is the absorptive capacity of the superabsorbent material.

Two liters of 0.9% by weight sodium chloride in distilled water is poured into a tray having dimensions 24 cm x 30 cm x 5 cm. The liquid filling height should be about 3 cm.

The teabag pouch has dimensions 6.5 cm x 6.5 cm and is available from Teekanne in Düsseldorf, Germany. The pouch is heat sealable with a standard kitchen plastic bag sealing device (e.g. VACUPACK2 PLUS from Krups, Germany).

The teabag is opened by carefully cutting it partially, and is then weighed. A 0.200g +/- 0.005g sample of the superabsorbent material is placed in the teabag. The teabag is then closed with a heat sealer. This is called the sample teabag. An empty teabag is sealed and used as a blank.

The sample teabag and the blank teabag are then laid on the surface of the saline solution, and submerged for about 5 seconds using a spatula to allow complete wetting (the teabags will float on the surface of the saline solution but are then completely wetted). The timer is started immediately.

After 20 minutes soaking time the sample teabag and the blank teabag are removed from the saline solution, and placed in a Bauknecht WS130, Bosch 772 NZK096 or equivalent centrifuge (230 mm diameter), so that each bag sticks to the outer wall of the centrifuge basket. The centrifuge lid is closed, the centrifuge is started, and the speed increased quickly to 1,400 rpm. Once the centrifuge has been stabilised at 1,400 rpm the timer is started. After 3 minutes, the centrifuge is stopped.

The sample teabag and the blank teabag are removed and weighed separately.

The Teabag Centrifuge Capacity (TCC) for the sample of superabsorbent hydrogel-forming material is calculated as follows:

TCC = [(sample teabag weight after centrifuging) - (blank teabag weight after centrifuging) - (dry superabsorbent hydrogel-forming material weight)] ÷ (dry superabsorbent material weight).

It is essential for the current invention that materials exhibit a large "surface-to-mass ratio" in excess of at least 0.2g/m2. Suitable methods to determine surface-to-mass ratios are well described in prior art, such as in US 5.328.935.

BET SURFACE AREA TO UNIT MASS

The specific surface area to unit mass ($m^2$/g) of the high surface to mass ratio materials is determined using the Brunauer-Emmet-Teller (BET) gas adsorption method. This method involves adsorbing a monolayer of a gas (Nitrogen ($N_2$)) on a known mass of a sample at liquid nitrogen temperatures. The adsorbed $N_2$ is then desorbed by raising the temperature of the sample (thermal desorption) and detected by a thermal conductivity detector (TCD) whose output is connected to an integrating recorder. The peak area of the desorbed $N_2$ is thus known. Replicate desorption peaks are recorded for each sample, the average of which is the signal area (A). After the sample analysis, the instrument response ($A_{cal}$) is determined by injecting known amounts ($V_c$) of Nitrogen gas (99.99%+) into the system and the instrument response is recorded via the integrating recorder. $A_{cal}$ is the average of the several instrument responses obtained upon injecting the known amounts of $N_2$. The A, $A_{cal}$ and $V_c$ values are then used to determine the specific surface area of the sample using the multi-point BET calculation.

The specific equipment used for these analyses is obtainable from the Quantachrome Corporation (Syosset, N. Y.) and consists of the Quantector Outgassing Station, the Flow Controller, and the Quantasorb Jr. Sample Analysis Unit. These instruments are used as described in the operating manuals for the Quantasorb Junior [®] Sorption System, 2/19985, incorporated herein by reference. Various specific $N_2$-Helium mixtures obtained by mixing pure $N_2$ and pure helium via the Flow Controller are used as the adsorbate gas.

0.75 grams +/- 0.05 grams of the sample is weighed into the glass sample cell (about 2.5 ml) of the apparatus. The glass cell containing the sample is then placed into the gas flow of the instrument. The samples are outgassed with a 30 ml/min Helium flow using the Quantector for a time sufficient to remove any gases other than Helium from the sample, typically a minimum of 4 hours. After outgassing, the gas flow is changed to a specific $N_2$-Helium gas mixture. The glass sample cell is immersed in liquid Nitrogen and allowed to reach equilibrium. An adsorption curve is generated. The adsorbed $N_2$ generates a desorption curve and a peak value (used to calculate the signal area (A)). Adsorption/desorption measurements are performed on each sample using different $N_2$-Helium gas mixtures.

The specific surface area Sg is calculated as follows:

$$S_g = S_t/W;$$

wherein W is the weight of the sample and $S_t$ equals $X_m (6.02 \times 10^{23}) A_{cs}$,

wherein $A_{cs}$ is the adsorbate cross sectional area. For $N_2$, $S_t$ becomes $X_m$ $(3.483 \times 10^3)$ $m^2$; wherein $X_m$ equals $1/(S+I)$. S is slope and I is the Y-intercept of the plot of $1/X \{P_o/P) - 1\}$ versus $P/P_o$.

In calculating the x and y values for the above plot, X equals $(A)X_c/(A)_{cal}$. A is the signal area; $A_{cal}$ is the calibration area; and $X_c$ equals $P_a M_a V_c/6.235 \times 10^4 T$. $P_a$ is the ambient pressure; $M_a$ is the molecular weight of the adsorbate which for $N_2$ becomes 28.0134; $V_c$ is the calibration volume; and T is the ambient temperature in K. P is the partial pressure of the absorbate; and $P_o$ the saturated vapor pressure, equals $P_g + P_a$; wherein $P_g$ is the vapor pressure (above ambient) and $P_a$ is the ambient pressure.

Creation of materials having such high surface to mass-ratios can start from forming web structures out of superabsorbent fibres with relatively fine thicknesses of about $20\mu m$ or less such as described in EP-B-0 565 606, or by finely grinding particulate materials and reconstituting these into macrostructures which remain essentially intact upon wetting, which is described in US patent 5 124 188.

A preferred execution is to start from superabsorbent polymer foams such as described in US 5.338.766 assigned to van Phan being made according to the teachings of US 5.328.935, also assigned to van Phan: Approximately 250 grams of aqueous (50% by weight ) acrylic acid are 75% neutralized to sodium acrylate using 0.1N NaOH. To avoid the formation of polyacrylic acid, the neutralization is performed by gradually adding the NaOH to the acrylic acid solution with gently mixing and cooling (with dry ice).

A reaction mixture is prepared as follows:

200 grams of the above prepared aqueous sodium acrylate solution; 0.50 grams $N_2N'$ -methylbisacrylamide; 1.3 grams V-50 (2,2' -azobis (2-amidinopropane) dihydrochloride, available from Wako Chemicals USA, Inc.); and 20 grams PEG-600 (polyethylene glycol having a weight average molecular weight of about 600, available from the Union Carbide Co. of Danbury, Conn) are added to a 1 liter glass reactor fitted with temperature and pressure controls and a high shear mixing apparatus (e.g., an "Ultra Turray" mixer, available from the Tekmar Company of Cincinnati, Ohio). The reactor is at ambient temperature (about 22°C) and pressure (about 1 atm). A mixture of 60 grams of Freon 1,1,2 (1,1,2-trichlorotrifluoroethane, available from Aldrich Chemical of Milwaukee, Wisc.); 3.5 grams SPAN®20 (sorbitan monolaurate, available from Aldrich Chemical) and 6.5 grams TWEEN ® 20 (ethoxylated sorbitan monolaurate, available from Aldrich Chemical) is then added to the reactor. This latter mixture is prepared in advance by adding the components to a reactor similar to the one described above and mixing well.

The reaction mixture is mixed at about 850 rpm for about 10 minutes to stably disperse the Freon 1,1,2. The mixing apparatus is then removed from the dispersion. A foam product is formed by increasing the reactor temperature to 60°C and maintaining the temperature at 60°C for about 1 hour; followed by increasing the temperature to 80°C and maintaining it at 80°C for about 30 minutes; followed by increasing the temperature to 120°C and maintaining it at 120°C for about 30 minutes. The reactor is then cooled to about ambient temperature (about 22°C).

A mixture of 5 grams of glycerol and 25 grams of isopropyl alcohol is added to the foam in the reactor to impregnate the foam with the mixture. The temperature of the reactor is then increased to and maintained at 180°C for about 1 hour. The reactor is cooled to ambient temperature (about 22°C), after which the foam is removed from the reactor and openly placed in a humidified room (80% relative humidity) for 6 hours.

When having such high surface to mass ration materials, one aspect of the current invention aims at providing such materials in a collapsed form. "Collapsed" means in the context of this invention, that the pore void volumes are minimized, possibly to the extent, that the porous structures are not necessarily readily apparent. However, whilst pore walls which can be formed by the fibres or particles or struts may be in direct contact with each other in the collapsed form (and hence the pore between the respective walls or struts essentially disappears or at least reduces its pore volume significantly), the surfaces remain essentially intact and do not coalesce together. Then, an expanded structure with comparable pore sizes as the comparative uncompressed structure can be reached upon wetting - e.g. by body fluids during use.

Without being bound by the theory, it is believed, that in this aspect of the invention residual amounts of plasticiser in the materials (such as residual moisture or glycerol) provide contractive forces, such as through hydrogen bonds, which overcome the restoring forces of the elastic deformation of the foam cells. Upon an increase of the amount of water molecules, the contractive forces are weakened and allow the elastic forces to restore the open pore volumes. At this moment, however, these materials not only start swelling through the classical superabsorbent swelling (or hydrogelling) mechanisms, but also allow liquid to penetrate into the material through and into the opened pores, thus immediately providing the large relative surface area for quick transfer of fluid into the superabsorbent polymer network.

The collapsed superabsorbent materials according to the current invention can be in various forms or physical shapes, most importantly either in "free" or in "three-dimensional" form.

The term "free" generally refers to particulate structures of varying shape and size, such as can be obtained by cutting, chopping, grinding, or agglomerating precursor particulate structures. Generally, such structures can be described by characteristic dimensions such as an "average particle size", but individual particles can vary quite significantly from neighbouring ones for example in size, shape, and so on.

A preferred execution refers to particulate structures of relatively large dimensions. Whilst such particles have general advantages such as reduced tendency for blocking distribution channels for fluids, or a reduced tendency of generating dust, the current invention provides a reduction of the generally occurring negatives of such materials, namely slow absorption - which is compensated by the increase of "internal" surface- , or "pockmarking" or unpleasant feel of hard particles by the wearer - which can being compensated by increased softness characteristics of the materials according to the present invention as discussed below.

However, for too large (expanded) particulate structures, there is a risk that the fluid transport from the surrounding material might not be adequate, such that collapsed materials should not exceed about 300μm in diameter.

Another preferred form of the materials according to the present invention is a "three-dimensional" form, such as sheets, stripes, and the like. Such structures have one dimension (i.e. length in the case of stripes) or two dimensions (i.e. length and width in the case of sheets) significantly larger than the thickness or caliper of the material, especially in the collapsed state. Further and in contrast to the particulate structures with generally stochastical distribution of shape and size, the dimensions are essentially the same for at least a great part of the structures (i.e. the dimensions and shapes varies only in relatively narrowly defined limits for essentially all structures for one specific application). Such structures can be cut from a "slab" of reacted foam material, or can be reacted in molds being filled with reacting foam, or can be applied in a form of a continuous layer of foam on to a carrier, which can be removed from the sheet like foam when the reacting foam has reached sufficient integrity or which can remain with the foam and be an element of the final absorbent article.

Another preferred execution of the current invention is to use superabsorbent fibres which are comprised in webs, which can be formed by a wide variety of conventional web forming techniques, such as carding to create nonwovens, or air laying, or the like. The webs can be bonded by any conventional means, such as through resin bonding by addition of suitable binders, or entanglement, such as with needles punching and entwining the fibres into a web. A preferred execution of such webs includes a low percentage of about 10% of polymeric fibres, which melt at a lower temperature than the superabsorbent fibres. Subsequent heat induced melting of these polymers is then providing sufficient strength to such webs, without unnecessary hindering swelling of the fibres. Suitable webs can be received according to the teachings of Noel in EP-B-0 565 606, assigned to Noel et al. by using 90% by weight of Fibersorb type 7200 fibres of Camelot Co., US, and 10% by weight of ES-E-WA 3.3.dTex fibres of DANAKLON AS, Denmark, and airlaying a structure of 140g/m2 with subsequent through air-bonding.

If starting from such high porosity structures, it is an essential feature of this invention to collapse these to sufficiently high densities without majorly impacting on the high specific surface values, and also not on other absorbent properties such as absorbent capacity, speed and the like. This can be achieved by a number of different ways.

In case of starting from essentially sheet like structures, collapsed structures according to the present invention can be made by placing such sheets into a conventional press, such as a hydraulic or mechanical press. In case of essentially endless structures, these can be collapsed by compressing the materials through a pair of compression (or calender) rolls. Tight subsequent winding can further minimize the tendency of a certain degree of "spring back", optionally with use of separating sheets between two adjacent layers on the rolls. Also, the resulting structure could be transformed into a particulate structures according to the invention, such as through conventional techniques such as grinding, chopping and the like.

Such processes apply particularly when starting from foamed structures, but are evenly applicable when starting from webs such as nonwovens comprising superabsorbent fibres.

Similar processes can be applied when starting from materials in the particulate form when being expanded which are then compressed. There, however, two effects have to be considered: First, the collapse of the pores as described in the sheet like structures, i.e. the intra-particle compression. Second, the compression of the particles into inter-particle voids by a deformation of the macroscopic particle shape. This second effect is not desired for superabsorbent materials exhibiting a relatively low gel strength or stiffness, as - even under only moderate in-use pressures - such materials would not open the inter-particle voids, even if the intra-particle pores are opened.

Other ways to create such collapsed structures also fall within the scope of this invention, such as collapse through evaporative moisture removal, whereby care must be taken that the structure is not overdried, vacuum suction dewatering and the like.

For any of these process options, further aids such as moisture, or plasticiser might be added during the compression step as to optimize the properties of the resulting structure with regard to resiliency and/ or softness.

Suitable plasticizers include water, high molecular weight hydrophilic organic solvents (e. g., glycerol; 1,3-propanediol; or ethylene glycol), or polymeric solutions (e.g., polyvinyl alcohol or polyethylene glycol), or mixtures thereof. The plasticizer can be applied in a number of different ways including spraying, coating, atomizing, immersing, or dumping the solution onto the structure. Alternatively, in the case of water, the plasticizer may be added via placing the structure into a high humidity environment (e.g., greater than 70% relative humidity).

The resulting structures will still be essentially dry, meaning that they have a moisture content of less than 50% by weight, preferably less than 20% by weight of the total structure.

Overall, volume reduction (collapse ratio) can be measured by comparing the volume of the structure prior and after the compression, or by measuring the volume of the (essentially dry) structure before wetting and of the re-dried structure, such as achieved by wetting the structure with a known amount of fluid to open up the cell pores, and then drying the structure without applying external pressure e.g. in an oven for 3 hrs at 105C.

This can be applied to three-dimensional structures (including webs comprising superabsorbent fibres), or to particulate structures.

Whilst the ability to remain permanently compressed until wetting is the most essential property according to the present invention, sheet like structures according to the present invention can have particularly beneficial softness properties. Whilst softness is generally a subjective measure there are methods describe to quantitatively assess this property:

DETERMINATION OF FLEXIBILITY

Flexibility of sheet-like structures, e. g. made of foams or of fibrous webs, can be quantified by referencing a test procedure which is a modification of the ASTM D 3574-86 test utilizing a sample which is 7 cm x 0.8 cm x 0.8 cm and which has been saturated to its equilibrium absorbent capacity (i.e. soaked in Synthetic Urine or 0.9% saline solution for about 15 minutes). The saturated strip is bent around a 0.8 cm diameter cylindrical mandrel at a uniform rate of 1 lap in 5 seconds until the ends of the strip meet. The sample is considered flexible if it does not tear or break during this test, i.e., if it passes one bending cycle.

It is important that the cutting process used to make the strip samples does not introduce edge defects in the strip. The strips of the requisite size should be cut using a sharp reciprocating knife saw. Use of this or an equivalent type of sharp cutting device serves to substantially eliminate sample edge flaws and edge densification effects which could have adverse impact on the accuracy of certain of the measurements made in carrying out the test procedure. In addition, caliper of thickness measurements should be made when the absorbent structure sample is under a confining pressure of 350 Pa (0.05 psi).

A further requirement is a good absorbency or "superabsorbency" of the material to be suitable for the present invention.

On one side, this is a quantitative terms for the equilibrium absorbent capacity, and materials used according to the present invention have not less than 15 grams of absorbed fluid per gram of material in the Teabag Retention Capacity Test.

On the other side it is a qualitative description of the absorption mechanisms, whereby the predominant part of the absorbed fluid is - at least at equilibrium conditions - absorbed into the polymeric network of the structure (and not only hold in pores).

Absorbent structures preparation

Generally, materials according to the present invention can be incorporated into absorbent structures by using conventional approaches well known to the man skilled in the art and explained above.

Specifically in the case of particulate materials, mixing of the compressed superabsorbent granules with fibrous materials such as cellulosic fibres can be applied, or laminates of such particles with carriers like tissues can be used, or fluid stable sheet-like aggregates.

In case of defined three-dimensional forms, sheets, sheet-like webs, stripes, rods, or other essentially endless structures these can be introduced into absorbent structures using for example the materials in roll-form and cutting the length as desired, for example covering the full length of the absorbent structure. It also can be in specific regions of the absorbent structure only, using for example the so called "cut-and-slip" process. Also precut sheets can be used to be introduced into absorbent structures by generally known techniques.

**Claims**

1. A porous material for absorption of aqueous body fluids comprising superabsorbent polymer, which is capable of swelling upon absorption of aqueous liquids, and which forms a porous structure having a surface-to-mass ratio of at least 0.2m2/g;
   characterized in that
   the pores of said porous structure are collapsed to less than a third of their expanded volume prior upon wetting with said aqueous body liquids;
   and in that the pores expand upon wetting with aqueous liquids.

2. A porous material according to claim 1,
   further characterized in that
   the expansion of the pores is non-isotropical and in one dimension significantly more than in at least one other dimension.

**3.** A superabsorbent material according to claims 1 or 2,
further characterized in that
it is flexibilized by means of a plasticiser material.

**4.** A superabsorbent material according to claim 3,
further characterized in that the plasticiser comprises water and/or glycerol.

**5.** A superabsorbent material according to claim 1 to 4,
further characterized in that it has in the collapsed state a density of between 0.05g/cm3 and 1.6g/cm3.

**6.** A superabsorbent material according to claims 1 to 5,
further characterized in that it has in the collapsed state a moisture content of between 4% and 30% on a dry basis.

**7.** A superabsorbent material accordant to claims 1 to 6,
further characterized in that
the pores in the expanded state have a pore size of less than 100$\mu$m, preferably 5 to 100$\mu$m.

**8.** A superabsorbent material according to claims 1 to 7
further characterized in that
the superabsorbent polymer is in the form of a foam comprising a plurality of mutually connected struts of superabsorbent polymer material to form open cells in the expanded state,
and in that it is collapsed such that density is in the range between 0.05g/cm3 and 1.6 g /cm3.

**9.** A superabsorbent material according to claims 1 to 8,
further characterized in that
it forms a three-dimensional structure of at least 10 mm3 volume it has an extension into one or two dimensions of at least 10 times the extension into the third dimension.

**10.** A superabsorbent structure according to claim 9,
further characterized in that
the expansion of the pores is non-isotropical and in one dimension significantly more than in at least one other dimension.

**11.** A superabsorbent material according to claims 1 to 7
further characterized in that
it is made of a web comprising superabsorbent in fibrous form.

**12.** A superabsorbent material according to claims 1 to 11,
further characterized in that it forms a flexible sheet.

**13.** A superabsorbent material according to claims 1 to 13,
further characterized in that
it is in the form of particles consisting essentially of foamed superabsorbent.

**14.** A superabsorbent material according to claim 13,
further characterized in that
the collapsed particles have a size of less than 300$\mu$m.

**15.** A method for preparing a superabsorbent material, comprising the steps of

providing an unexpanded porous superabsorbent material
containing a fluid in its pores;
collapsing the pores of the material by removing fluid from the
pores of the uncollapsed material.

**16.** A method for preparing a superabsorbent material according to claim 15, whereby the collapsing the pores of the material is achieved by mechanical action on the material in at least one direction.

**17.** A method for preparing a superabsorbent material according to claim 15, whereby the collapsing the pores of the material is achieved by evaporative removal of fluids.

18. A method according to claim 17
    additionally comprising the step of addition of a plasticiser.

19. An absorbent structure comprising any materials according to claims 1 to 14.

20. A disposable absorbent article comprising an absorbent structure according to claim 19.

**Fig. 1**

**European Patent Office**

## EUROPEAN SEARCH REPORT

Application Number

EP 96 10 5021

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | US-A-5 338 766 (D.VAN PHAN ET AL.)<br><br>* column 3, line 39 - line 48 *<br>* column 30, line 39 - line 48 *<br>--- | 1-10,<br>12-20 | A61F13/15<br>A61L15/42<br>A61L15/60 |
| D,Y | WO-A-94 13704 (PROCTER & GAMBLE)<br><br>* page 5, line 19 - line 22 *<br>* page 5, line 29 - page 6, line 18 *<br>* page 7, line 12 - line 13 *<br>* page 9, line 23 - page 10, line 3 *<br>* page 12, line 15 - line 20 *<br>* page 13, line 23 - line 25 *<br>* page 16, line 3 - line 7 *<br>* page 19, line 27 - line 31 *<br>* page 22, line 18 - line 24 *<br>* page 50, line 1 - line 35 *<br>* page 51, line 34 - line 35 *<br>* page 52, line 5 - line 33 *<br>--- | 1-10,<br>12-20 | |
| D,A | US-A-5 328 935 (P.D.TROKHAN AND D.VAN PHAN)<br>* column 13, line 44 - line 52 *<br>* column 22, line 35 - line 52 *<br>--- | 1,3,18 | TECHNICAL FIELDS SEARCHED (Int.Cl.6)<br><br>A61F<br>A61L |
| A | US-A-3 653 383 (R.G.WISE)<br><br><br><br>* column 3, line 65 - column 4, line 3 *<br>--- | 1,5,8,<br>12,<br>15-17,<br>19,20 | |
| A | US-A-5 260 345 (T.A.DESMARAIS ET AL.)<br><br>* column 39, line 60 - column 40, line 26 *<br>--- | 1,2,9,<br>10,15-17 | |
| A | EP-A-0 414 541 (ARCO CHEMICAL TECHNOLOGY)<br>* page 3, line 28 - line 45 *<br>----- | 11 | |

The present search report has been drawn up for all claims

| Place of search | Date of completion of the search | Examiner |
|---|---|---|
| THE HAGUE | 7 August 1996 | Nice, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)